# DEMANDE DE BREVET EUROPEEN

(11) **EP 4 635 449 A1**
(43) Date de publication de la demande: **22.10.2025**
(21) Numéro de dépôt: 24305613.2
(22) Date de dépôt: 19.04.2024
(51) Int. Cl.: A61F 2/00

(54) **IMPLANT TESTICULAIRE POUR L'AUGMENTATION DU VOLUME**

(71) Demandeur: Naxon.One, 75017 Paris (FR)
(72) Inventeur: ATHMANI, Bachir, 75017 Paris (FR); ABECASSIS, Marc, 75011 Paris (FR)
(74) Mandataire: Icosa

(57) **Abrégé**

La présente invention concerne un implant testiculaire, et plus particulièrement un implant testiculaire à destination des hommes.

## Description

### DOMAINE DE L'INVENTION

La présente invention concerne un implant testiculaire, et plus particulièrement un implant testiculaire à destination des hommes.

### ÉTAT DE LA TECHNIQUE

La taille et la forme des testicules chez l'homme peuvent être des sujets de complexe et d'inconfort. Par exemple, pour des raisons médicales, à la suite de blessures, ou d'une maladie, les testicules peuvent présenter une taille qui ne convient pas au patient, ce qui crée une gêne, en particulier dans l'intimité. Dans d'autres cas, pour des raisons esthétiques, certains hommes souhaitent augmenter la taille de leurs testicules.

Des implants ayant pour but d'augmenter la taille des testicules, par exemple décrits dans EP 3666226, sont connus. Ces implants répondent au besoin d'augmentation de taille des testicules mais présentent des désavantages lors de leur implantation. En effet, ces derniers sont difficiles à placer correctement. Cependant, un mauvais placement de l'implant peut créer un inconfort pour le patient et peut également atténuer l'effet esthétique de l'implant.

A plus forte raison, l'implantation d'implant testiculaire peut être liée à des complications éventuelles, telles que le déplacement ou la rotation de l'implant. Cela génère alors de l'inconfort.

Il existe donc un besoin d'amélioration de ces implants pour résoudre, au moins en partie, ces défauts.

### RÉSUMÉ

Implant testiculaire comprenant un corps d'implant de forme ovoïde s'étendant le long d'un axe, le corps d'implant présentant une face externe, une base, un logement, et une ouverture configurée pour s'ouvrir sur le logement
dans lequel le logement étant apte à accueillir un testicule humain, le testicule humain comprenant un bord postéro-supérieur reliée à l'épididyme et un bord antéro-inférieur libre faisant face au scrotum,
le corps d'implant étant configuré pour être placé entre le testicule et le scrotum, l'implant enveloppant le testicule en laissant libre l'épididyme, et
dans lequel le corps d'implant comprend un élément d'orientation sur sa face externe.

L'élément de guidage de l'implant permet au praticien posant l'implant de facilement repérer l'orientation de l'implant sans nécessiter de vision directe sur ce dernier. Par conséquent l'implant est plus simple à positionner correctement sur le patient.

Ainsi, le risque de mal positionner l'implant est diminué. Cela est particulièrement avantageux car si l'implant est mal positionné, alors l'esthétique de l'implant est significativement réduite.

Dans certains modes de réalisation, l'élément d'orientation est un repère visuel, typiquement un marquage coloré.

Dans certains modes de réalisation, l'élément d'orientation est une saillie.

Dans un autre mode de réalisation, l'élément d'orientation est une rainure creusée dans le corps d'implant.

Lorsque l'élément d'orientation est saillant, il est plus simple de l'orienter au toucher.

Dans certains modes de réalisation, l'élément d'orientation est longiligne.

Un tel élément d'orientation est plus simple à réaliser, ce qui rend la fabrication de l'implant plus simple.

Dans certains modes de réalisation, l'élément d'orientation s'étend dans un plan comprenant l'axe, sur la partie de la face externe configurée pour faire face au bord antéro-inférieur du testicule.

Lorsque l'élément d'orientation est placé de cette façon, ce dernier est moins visible lorsque l'implant est placé sur un patient. De plus, un tel placement de l'élément d'orientation augmente la stabilité de l'implant. En effet, l'élément d'orientation ainsi positionné prévient les mouvements indésirables tels que le basculement ou la rotation, garantissant ainsi que l'implant reste dans la position souhaitée après l'opération. Cela permet donc de maintenir l'intégrité esthétique de l'implant tout en minimisant le besoin d'interventions chirurgicales correctives.

Dans certains modes de réalisation, le corps d'implant est en silicone.

Le silicone est un matériau facilement accessible et est biocompatible. Ce matériau est donc particulièrement adapté pour une application d'implant.

Dans certains modes de réalisation, la hauteur de l'élément d'orientation selon l'axe est trois à quatre fois plus petite que la hauteur de l'implant testiculaire selon l'axe.

Une telle dimension est un compromis adapté entre discrétion de l'implant et facilité de placement pour le praticien.

Dans certains modes de réalisation, le corps d'implant présente une épaisseur, et dans lequel l'épaisseur du corps d'implant est maximale au niveau de la base du corps d'implant.

La surépaisseur au niveau de la base du corps d'implant permet de stabiliser l'implant, qui se positionnera naturellement sur sa base sous l'action de la gravité. L'implant s'aligne alors plus facilement le long de la direction de gravité.

Dans certains modes de réalisation, le corps d'implant comprend une pluralité d'éléments d'orientations sur la face externe.

La pluralité d'élément d'orientation permet d'orienter plus facilement l'implant.

Dans certains modes de réalisation, les chaque éléments d'orientation de la pluralité d'éléments d'orientation sont sécants.

Selon ce mode de réalisation, le point d'intersection des éléments d'orientation permet de fournir un repère supplémentaire au praticien, ce qui facilite davantage son positionnement sur le patient.

Dans certains modes de réalisation, les éléments d'orientation sont sécants au niveau de la base du corps d'implant.

Cette configuration est une alternative ou un complément de la surépaisseur à la base de l'implant. L'implant est donc plus facilement positionnable et est plus stable dans son alignement le long de la direction de gravité.

Dans certains modes de réalisation, le ou chaque élément d'orientation ont une largeur comprise entre 1 mm et 5 mm.

Une telle dimension est un compromis adapté entre discrétion de l'implant et facilité de placement pour le praticien.

Dans certains modes de réalisation, le corps d'implant comprend au moins deux orifices de fixation configurés pour accueillir un moyen de fixation de l'implant sur le testicule.

Les orifices de fixation permettent de suturer l'implant au testicule à l'aide d'un fil de suture passant au travers des orifices de fixation, ce qui renforce grandement sa stabilité. En effet, du fait de la suture, l'implant est maintenu au contact des testicules de façon plus sûre, tout en restreignant des éventuels mouvements de rotation et/ou de basculement de l'implant.

Dans certains modes de réalisation, le corps d'implant comprend un nombre pair d'orifices de fixation, et dans lequel la position de chaque orifice de fixation sur la face externe est une position symétrique par rapport à l'axe d'un autre orifice de fixation.

Cette configuration permet de répartir les points de fixation de façon homogène, ce qui renforce la stabilité de l'implant.

Dans certains modes de réalisation, les orifices de fixation présentent un diamètre compris entre 1 mm et 2 mm.

Un tel diamètre est adapté au passage aisé d'un fil de suture, tel qu'on peut les trouver dans le commerce. Par ailleurs, cela renforce la discrétion des orifices de fixation, ce qui contribue à l'aspect esthétique de l'implant.

Dans certains modes de réalisation, chaque orifice de fixation est distant, le long de la face externe, d'au moins 5 mm d'un autre orifice de fixation.

Dans cette configuration, la flexion du fil de suture est diminuée, ce qui renforce la robustesse de la suture, et par conséquent la stabilité de l'implant.

Dans certains modes de réalisation, la distance, le long de la face externe, entre un orifice de fixation et l'ouverture est plus petite que la distance, le long de la face externe, entre un orifice de fixation et la base du corps d'implant.

Dans certains modes de réalisation, la distance, le long de la face externe, entre un orifice de fixation et l'ouverture est au moins trois fois plus faible que la distance, le long de la face externe, entre un orifice de fixation et la base de la face externe.

Dans cette configuration, la fixation de l'implant sur le testicule s'opère sans laisser de bord libre, ce qui renforce la stabilité de l'implant et la robustesse de la fixation.

Le présent exposé concerne par ailleurs une méthode chirurgicale comprenant une anesthésie, une asepsie, une intervention chirurgicale dans laquelle un implant selon l'un des précédents aspects est implanté dans un patient et, optionnellement, des soins post-opératoires.

### BRÈVE DESCRIPTION DES FIGURES

La **Figure 1** représente schématiquement un implant testiculaire selon un premier mode de réalisation.
La **Figure 2** représente schématiquement l'implant testiculaire du mode de réalisation de la figure 1 disposé autour d'un testicule humain.
La **Figure 3** représente schématiquement une vue tronquée de l'implant testiculaire selon le mode de réalisation de la figure 1.
La **Figure 4** représente schématiquement un implant testiculaire selon un troisième mode de réalisation, comprenant une pluralité d'éléments d'orientation.
La **Figure 5** représente schématiquement un implant testiculaire selon un quatrième mode de réalisation, comprenant une pluralité d'éléments d'orientation sécants au niveau de la base du corps d'implant.

### DESCRIPTION DÉTAILLÉE

Des exemples de réalisation sont décrits dans le présent exposé. L'invention ne saurait être limitée à ces exemples.

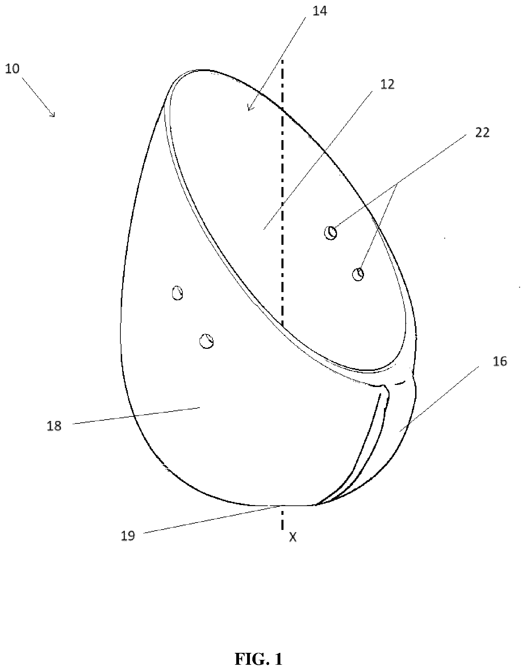

La figure 1 représente schématiquement un implant testiculaire 10 selon un mode de réalisation. L'implant 10 comprend un corps d'implant 18 de forme ovoïde s'étendant le long d'un axe X. En d'autres termes, l'implant présente une forme ovale tronquée, semblable à une coquille d'oeuf.

Le corps d'implant 18 présente une face externe, une base 19, un logement 12, et une ouverture 14 configurée pour s'ouvrir sur le logement 12.

Le corps d'implant 18 peut être prévu dans tout matériau biocompatible. Par exemple, le corps d'implant 18 peut être en silicone.

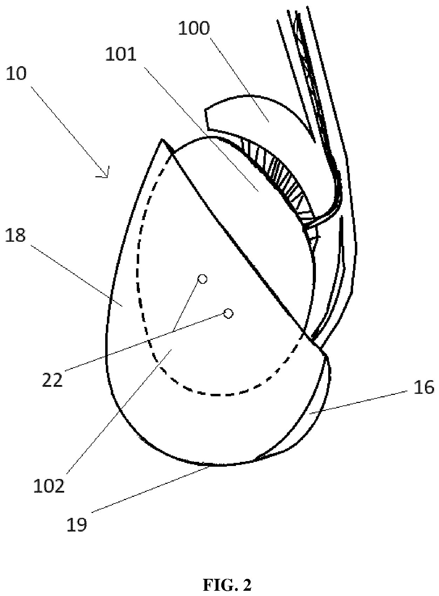

Le logement 12 est apte à accueillir un testicule humain. En référence à la figure 2, le testicule humain comprend un bord postéro-supérieur 101 reliée à l'épididyme 100 et un bord antéro-inférieur 102 libre, qui fait face au scrotum. On note par ailleurs que, lorsque l'implant 10 est disposé autour d'un testicule d'un patient se tenant debout, la direction X correspond à la direction de la gravité.

Figure 2 représente schématiquement l'implant testiculaire du mode de réalisation de la figure 1 disposé autour d'un testicule humain. Comme cela s'observe sur la figure 2, l'implant 10 est configuré de sorte que le corps d'implant soit placé entre le testicule et le scrotum, l'implant enveloppant le testicule en laissant libre l'épididyme 100.

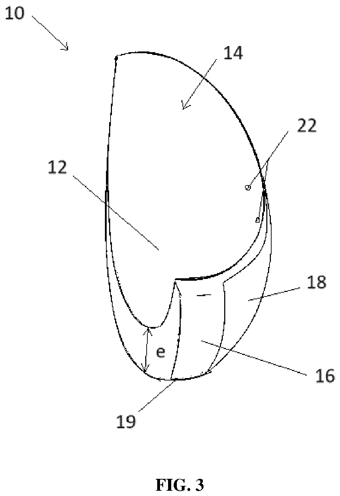

La figure 3 représente schématiquement une vue tronquée de l'implant testiculaire selon le mode de réalisation de la figure 1. Comme cela est représenté sur la figure 2, le corps d'implant 18 peut présenter une épaisseur e variable. Dans d'autres exemples, l'épaisseur e du corps d'implant 18 peut être constante.

Lorsque l'épaisseur e du corps d'implant 18 est variable, l'épaisseur e peut avantageusement être maximale au niveau de la base 19 du corps d'implant 18.

Par ailleurs, le corps d'implant 18 comprend au moins un élément d'orientation 16 sur sa face externe. L'élément d'orientation 16 peut être une saillie, une rainure ou tout autre type de forme reconnaissable au toucher ou visuellement. Par exemple, l'élément d'orientation peut être un repère coloré. D'autre part, l'élément d'orientation 16 peut être longiligne, incurvé, continu ou non continu.

Dans l'exemple de réalisation de la figure 1, l'élément d'orientation 16 s'étend sur la face externe du corps d'implant 18, dans un plan comprenant l'axe X, sur la partie de la face externe qui est configurée pour faire face au bord antéro-inférieur 102 du testicule. En d'autres termes, l'élément d'orientation 16 est configuré pour s'étendre sensiblement le long de la direction de la gravité lorsque l'implant 10 est disposé sur un patient se tenant debout. r

L'élément d'orientation 16 peut s'étendre sur la face externe du corps d'implant 18 jusqu'à l'ouverture 14. L'élément d'orientation 16 peut, d'autre part, s'étendre sur la base 19 du corps d'implant 18.

Lorsque l'élément d'orientation 16 est une rainure ou une saillie, l'élément d'orientation 16 peut présenter une largeur comprise entre 1mm et 5mm et une épaisseur comprise entre 1mm et 3mm. De telles dimensions sont suffisantes pour que l'élément d'orientation 16 soit palpable pour le praticien, tout en étant suffisamment discrètes pour ne pas générer d'inconfort pour le patient et pour ne pas dénaturer l'esthétique de l'implant 10.

D'autre part, la hauteur de l'élément d'orientation 16 selon l'axe X peut être trois à quatre fois plus petite que la hauteur de l'implant testiculaire selon ledit axe X. En d'autres termes, lorsque l'implant 10 repose sur sa base 19, et que l'axe X est confondu avec la direction de la gravité, l'élément d'orientation 16 s'étend sur une distance, selon l'axe X, trois à quatre fois plus petite que la hauteur totale de l'implant 10 selon ledit axe X. Par exemple, considérant que l'implant 10 s'étend, le long de l'axe X, d'une partie inférieure, correspondant à la base 19 à une partie supérieure, l'élément d'orientation peut s'étendre uniquement sur le tiers inférieur du corps d'implant 18.

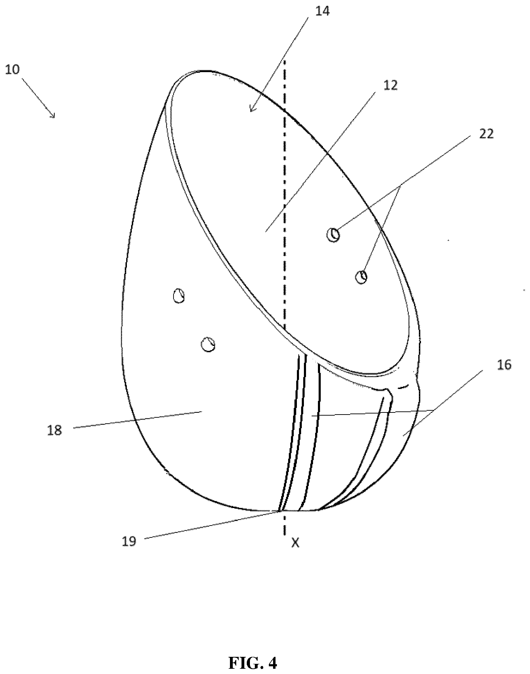

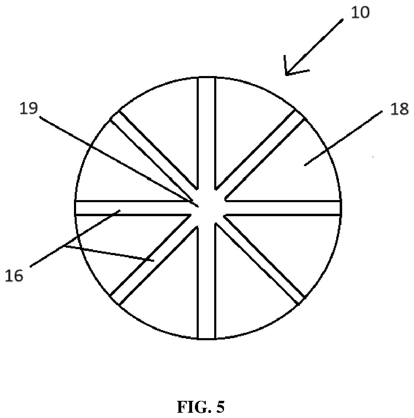

Comme cela est représenté sur la figure 4, le corps d'implant peut comprendre une pluralité d'éléments d'orientation, chaque élément d'orientation 16 peut présenter les caractéristiques précédemment décrites. Par ailleurs, comme cela est représenté sur la figure 5, les éléments d'orientation 16 de la pluralité d'éléments d'orientation peuvent avantageusement être sécants, et encore plus avantageusement être sécants au niveau de la base 19 du corps d'implant 18.

Au moins deux orifices de fixation 22 peuvent être prévus sur le corps d'implant 18. Ces orifices de fixation 22 peuvent être circulaires et présenter un diamètre compris entre 1mm et 2mm. Dans d'autres exemples, les orifices de fixation 22 peuvent être des encoches, des languettes ou une combinaison de ces éléments, configurées pour créer des points de fixation.

Les orifices de fixation 22 sont configurés pour aménager un moyen de fixation de l'implant 10 au testicule, par exemple un fil de suture.

Avantageusement, les orifices de fixation sont répartis sur la face externe du corps d'implant 18. Par exemple, le corps d'implant 18 peut comprendre un nombre pair d'orifices de fixation 22, la position de chaque orifice de fixation 22 sur la face externe du corps d'implant 18 étant une position symétrique par rapport à l'axe X d'un autre orifice de fixation 22. En d'autres termes, les orifices de fixation 22 sont disposés de part et d'autre de l'axe X.

D'autres répartitions des orifices de fixation 22 peuvent être prévus. Par exemple, comme cela est représenté sur la figure 1, les orifices de fixation 22 peuvent être prévus par paire, chaque orifice de fixation 22 de la paire d'orifice de fixation pouvant être distants d'au moins 5 mm le long de la face externe du corps d'implant.

De façon plus générale, les orifices de fixation 22 peuvent être distants d'au moins 5 mm le long de la face externe du corps d'implant.

Les orifices de fixation 22 peuvent être placés proche de l'ouverture 14, de sorte que la distance, le long de la face externe du corps d'implant 18, entre un orifice de fixation 22 et l'ouverture 14 est plus faible que la distance, le long de la face externe, entre un orifice de fixation 22 et la base 19. Cette distance peut être, par exemple, au moins trois fois plus faible.

Le présent implant 10 peut être implanté chez un patient selon une méthode chirurgicale comprenant une anesthésie, une asepsie, une intervention chirurgicale et des soins post-opératoires. Le patient est préférentiellement installé en position dorsale durant la méthode chirurgicale, et plus particulièrement pendant l'intervention chirurgicale.

En premier lieu, le patient est anesthésié par une anesthésie générale ou locale. Dans le cas d'une anesthésie locale, le patient est anesthésié au moins au niveau des testicules.

Ensuite, avant l'intervention chirurgicale, une asepsie est réalisée, afin de maintenir un état stérile pendant toute la durée de l'intervention chirurgicale et éviter toute contamination du patient.

L'intervention chirurgicale se déroule de la façon suivante. Tout d'abord, une incision cutanée verticale unique est réalisée sur le raphé médian scrotal, par exemple à l'aide d'une lame froide n°15. L'incision peut être effectuée en s'appuyant sur le billot testiculaire mis en tension entre deux doigts du chirurgien et peut typiquement s'étendre sur 4 à 5 cm. Ainsi, l'incision est suffisamment large pour permettre l'extériorisation atraumatique du testicule, et pour permettre la réintroduction intra-scrotale dudit testicule attaché à l'implant 10 à la fin de l'intervention chirurgicale.

Ensuite, le testicule, dans sa tunique vaginale, est extériorisé. Les différentes couches recouvrant la tunique vaginale, telles que le muscle dartos, la tunique celluleuse sous-cutanée, le fascia spermatique externe et la tunique fibreuse profonde, sont incisées, isolées puis électrocoagulées. Ces actes peuvent, par exemple, s'opérer à l'aide de ciseaux froids et/ou à l'aide un bistouri électrique.

La tunique vaginale est ouverte, de sorte à libérer le testicule et l'épididyme, qui sont alors mobilisés et extériorisés. A ce stade une inspection et une palpation complète peuvent être opérées.

Le testicule est ensuite introduit à l'intérieur du logement 12 de l'implant 10 par l'ouverture 14 de l'implant 10. L'implant 10 est alors positionné de sorte à envelopper partiellement le testicule en laissant l'épididyme libre. L'orientation de l'implant est vérifiée par le chirurgien grâce à l'élément d'orientation 16 palpable sur l'implant 10.

L'implant 10 peut être fixé au testicule à l'aide des orifices de fixation 22 optionnellement prévus sur l'implant 10. Un fil de suture, par exemple un fil monobrin non résorbable 4/0, est alors passé au travers des orifices de fixation de sorte à suturer l'implant 10 au testicule. Avantageusement, une le passage des sutures à travers l'albuginée est minimisé, restant superficiel, de sorte à limiter voire éviter tout dommage à la pulpe testiculaire sous-jacente.

Avant de réintroduire le testicule enveloppé par l'implant 10 dans le scrotum, une préparation atraumatique de la loge est effectuée au doigt. Cette préparation assure la création d'une loge adéquate pour recevoir le testicule et son implant sans risque de compression ou de torsion.

Le testicule enveloppé par l'implant 10 est ensuite introduit dans la loge ainsi crée, la position de l'implant 10 est vérifiée, et les parties incisées sont suturées. Le muscle dartos peut par exemple être refermé avec un surjet ou des points séparés utilisant un fil tressé à résorption lente 3/0. Enfin, la peau scrotale est refermée avec des points séparés de fil monobrin à résorption rapide 3/0.

Les soins post-opératoires peuvent comprendre l'application d'un pansement sec sur la cicatrice, par exemple pendant quelques jours, puis l'exposition à l'air de la cicatrice. Optionnellement, un pansement compressif peut être employé.

## Revendications

1. Implant testiculaire (10) comprenant un corps d'implant (18) de forme ovoïde s'étendant le long d'un axe (X), le corps d'implant (18) présentant une face externe, une base (19), un logement (12), et une ouverture (14) configurée pour s'ouvrir sur le logement (12),
a. dans lequel le logement (12) étant apte à accueillir un testicule humain, le testicule humain comprenant un bord postéro-supérieur (101) reliée à l'épididyme (100) et un bord antéro-inférieur (102) libre faisant face au scrotum,
b. le corps d'implant (18) étant configuré pour être placé entre le testicule et le scrotum, l'implant (10) enveloppant le testicule en laissant libre l'épididyme (100), et
c. dans lequel le corps d'implant (18) comprend un élément d'orientation (16) sur sa face externe.

2. Implant testiculaire selon la revendication 1 dans lequel l'élément d'orientation (16) est une saillie.

3. Implant testiculaire selon l'une des revendications 1 ou 2, dans lequel l'élément d'orientation (16) est longiligne.

4. Implant testiculaire selon l'une des revendications 1 à 3, dans lequel l'élément d'orientation (16) s'étend dans un plan comprenant l'axe (X), sur la partie de la face externe configurée pour faire face au bord antéro-inférieur (102) du testicule.

5. Implant testiculaire selon l'une des revendications 1 à 4 dans lequel le corps d'implant (18) est en silicone.

6. Implant testiculaire selon l'une des revendications 1 à 5, dans lequel la hauteur de l'élément d'orientation (16) selon l'axe (X) est trois à quatre fois plus petite que la hauteur de l'implant testiculaire (10) selon l'axe (X).

7. Implant testiculaire selon l'une des revendications 1 à 6, dans lequel le corps d'implant (18) présente une épaisseur (e), et dans lequel l'épaisseur (e) du corps d'implant (18) est maximale au niveau de la base du corps d'implant (19).

8. Implant testiculaire selon l'une des revendications 1 à 7, dans lequel le corps d'implant (18) comprend une pluralité d'éléments d'orientations sur la face externe.

9. Implant testiculaire selon la revendication 8, dans lequel les éléments d'orientation (16) de la pluralité d'éléments d'orientation sont sécants.

10. Implant testiculaire selon la revendication 9, dans lequel les éléments d'orientation (16) sont sécants au niveau de la base (19) du corps d'implant.

11. Implant testiculaire selon l'une des revendications 1 à 10, dans lequel le ou chaque élément d'orientation (16) ont une largeur comprise entre 1 mm et 5 mm.

12. Implant testiculaire selon l'une des revendications 1 à 11, dans lequel le corps d'implant (18) comprend au moins deux orifices de fixation (22) configurés pour accueillir un moyen de fixation de l'implant (10) sur le testicule.

13. Implant testiculaire selon la revendication 12, dans lequel le corps d'implant (18) comprend un nombre pair d'orifices de fixation (22), et dans lequel la position de chaque orifice de fixation (22) sur la face externe est une position symétrique par rapport à l'axe (X) d'un autre orifice de fixation (22).

14. Implant testiculaire selon l'une des revendications 12 ou 13, dans lequel les orifices de fixation (22) présentent un diamètre compris entre 1 mm et 2 mm.

15. Implant testiculaire selon l'une des revendications 12 à 14, dans lequel chaque orifice de fixation (22) est distant, le long de la face externe, d'au moins 5 mm d'un autre orifice de fixation (22).
